# EUROPÄISCHE PATENTSCHRIFT

(11) **EP 3 560 528 B1**
(45) Veröffentlichungstag und Bekanntmachung des Hinweises auf die Patenterteilung: **03.08.2022**
(21) Anmeldenummer: 18169257.5
(22) Anmeldetag: 25.04.2018
(51) Int. Cl.: A61M 1/00

(54) **SPÜLSAUGER**
SUCTION IRRIGATION DEVICE
DISPOSITIF DE RINÇAGE ET D'ASPIRATION

(43) Veröffentlichungstag der Anmeldung: 30.10.2019
(73) Patentinhaber: Lazic Besitz GmbH & Co. KG, 78532 Tuttlingen (DE)
(72) Erfinder: LAZIC, Daniel, 78532 Tuttlingen (DE)
(74) Vertreter: Kohler Schmid Möbus Patentanwälte

(56) Entgegenhaltungen:
- DE-A1- 3 309 916
- US-A- 5 449 145
- US-A- 5 658 249
- US-A1- 2009 187 146

## Beschreibung

Die Erfindung betrifft einen Spülsauger aufweisend ein Spülsaugergehäuse mit einem Saugkanal, einem Spülkanal und einem Saugspülkanal sowie mit einem Ventilraum, in den die Kanäle jeweils münden bzw. abgehen, und einen im Ventilraum gegen die Kraft einer Rückstellfeder verschiebbar geführten, manuell betätigbaren Ventildruckknopf, der mindestens einen Dichtungsabschnitt zum Steuern der Verbindung des Saugspülkanals mit dem Saugkanal und/oder dem Spülkanal aufweist.

Ein derartiger Spülsauger ist beispielsweise durch DE 33 09 916 C2, DE 33 09 917 C2 oder DE 33 09 916 C2 bekannt geworden. Derartige Saugsauger finden beispielsweise für medizinische Zwecke Anwendung, so zum Reinigen von Wunden bei chirurgischen Eingriffen. Dabei soll während einer Operation entweder Flüssigkeit abgesaugt oder Spülflüssigkeit hinzugegeben werden. Über ein Ventil wird die Spülflüssigkeit und die Saugleistung zugeschaltet bzw. abgeschaltet. Ein solcher Saugsauger sollte billig sein und aus Kunststoffmaterial bestehen, da er nach einmaligem Gebrauch weggeworfen wird.

Bei dem aus DE 33 09 916 C2, DE 33 09 917 C2 oder DE 33 09 916 C2 bekannten Spülsauger ist der Ventildruckknopf aus einem steifen Kunststoff gefertigt und weist drei aufgesetzte, aus Elastomermaterial bestehende elastische Dichtungsringe auf, welche mit der Wandung des Ventilraums in Kontakt stehen. Die Rückstellfeder ist einerseits am Boden des Ventilraums und anderseits an der Unterseite des Ventildruckknopfes abgestützt.

Demgegenüber ist es eine Teilaufgabe der vorliegenden Erfindung, bei einem Spülsauger der eingangs genannten Art die Anzahl der Einzelteile zu reduzieren und dadurch die Montage zu vereinfachen und die Herstellungskosten zu senken.

Die Erfindung wird durch die Merkmale des unabhängigen Anspruchs definiert.

Die Teilaufgabe wird erfindungsgemäß dadurch gelöst, dass der Ventildruckknopf einschließlich des mindestens einen Dichtungsabschnitts einstückig aus einem gummielastischen Material, insbesondere aus Silikon (z.B. Silikonkautschuk bzw. Silikonelastomere), gebildet ist.

Bei einer besonders bevorzugten Ausführungsform weist der Ventildruckknopf eine zum Gehäuseboden des Ventilraums offene Bohrung mit einem Absatz, insbesondere Bohrungsgrund, auf, an dem die Rückstellfeder abgestützt ist.

Bei einer anderen Ausführungsform steht von einem Gehäuseboden des Ventilraums ein Führungsvorsprung hoch, auf dem sowohl der Ventildruckknopf als auch die Rückstellfeder geführt sind. In diesem Fall kann der Ventildruckknopf eine zum Gehäuseboden des Ventilraums offene, gestufte Sackbohrung mit einem kleineren und einem größeren Bohrungsdurchmesser sowie mit einem Absatz, insbesondere Ringabsatz, aufweisen, an dem die Rückstellfeder abgestützt ist, wobei der kleinere Bohrungsdurchmesser dem Durchmesser des Führungsvorsprungs und der größere Bohrungsdurchmesser mindestens dem Außendurchmesser der Rückstellfeder entspricht.

In beiden Ausführungsformen ist es aus Stabilitätsgründen vorteilhaft, wenn der Absatz in einem breiten Mittel- oder Betätigungsabschnitt des Ventildruckknopfs angeordnet ist, damit der Ventildruckknopf an seinem Dichtende möglichst wenig verformt wird.

Der Ventildruckknopf weist bevorzugt einen aus dem Spülsauergehäuse herausragenden Betätigungsabschnitt, einen zylindrischen Schaft und dazwischen einen zylindrischen Mittelabschnitt auf, gegenüber dem der Betätigungsabschnitt und der Schaft jeweils radial nach innen zurückversetzt sind. Am Schaft ist beispielsweise ein Dichtungsabschnitt in Form einer radial nach außen vorstehenden, ringförmigen Dichtlippe einstückig angeformt, welcher die Verbindung des Saugspülkanals mit dem Spülkanal in einer ersten Ventilstellung des Ventildruckknopfs sperrt und in einer zweiten Ventilstellung des Ventildruckknopfs freigibt. Am Mittelabschnitt ist beispielsweise ein Dichtungsabschnitt in Form einer radial nach außen vorstehenden, ringförmigen Dichtlippe einstückig angeformt, welcher die Verbindung des Saugspülkanals mit dem Saugkanal in der ersten Ventilstellung des Ventildruckknopfs freigibt und in der zweiten Ventilstellung sperrt.

Der Ventilraum ist gestuft mit einem ersten Ventilraum mit größerem Durchmesser, in den der Saugkanal und der Saugspülkanal münden bzw. abgehen, und mit einem zweiten Ventilraum mit kleinerem Durchmesser, in den der Spülkanal mündet, ausgebildet. Der Schaftdurchmesser ist bevorzugt kleiner als der Durchmesser des zweiten Ventilraums, und der am Schaft angeformte Dichtungsabschnitt ist in der ersten Ventilstellung des Ventildruckknopfs auf der dem ersten Ventilraum zugewandten Seite des in den zweiten Ventilraum mündenden Spülkanals und in der zweiten Ventilstellung auf der dem ersten Ventilraum abgewandten Seite des in den zweiten Ventilraum mündenden Spülkanals angeordnet. Besonders bevorzugt weist der Saugkanal eine nach außen offene Saugregulierungsöffnung auf, die vorteilhaft auf der gleichen Gehäuseseite wie der Ventildruckknopf angeordnet ist. Je nach gewünschter Saugleitung wird die Saugregulierungsöffnung vom Bediener unterschiedlich weit freigegeben.

Weitere Vorteile der Erfindung ergeben sich aus der Beschreibung, den Ansprüchen und der Zeichnung. Ebenso können die vorstehend genannten und die noch weiter aufgeführten Merkmale je für sich oder zu mehreren in beliebigen Kombinationen Verwendung finden. Die gezeigten und beschriebenen Ausführungsformen sind nicht als abschließende Aufzählung zu verstehen, sondern haben vielmehr beispielhaften Charakter für die Schilderung der Erfindung.

Es zeigen:
- Fign. 1a, 1b: einen Längsschnitt einer ersten Ausführungsform des erfindungsgemäßen Spülsaugers mit einem Ventildruckkopf, der in Fig. 1a in einer Ventilausgangsstellung und in Fig. 1b in einer Ventilendstellung gezeigt ist;
- Fign. 2a, 2b: eine Seitenansicht (Fig. 2a) und eine Querschnittansicht (Fig. 2b) des in Fign. 1a, 1b gezeigten Ventildruckknopfs;
- Fign. 3a, 3b: einen Längsschnitt einer zweiten Ausführungsform des erfindungsgemäßen Spülsaugers mit einem Ventildruckkopf, der in Fig. 3a in einer Ventilausgangsstellung und in Fig. 3b in einer Ventilendstellung gezeigt ist; und
- Fign. 4a, 4b: eine Seitenansicht (Fig. 4a) und eine Querschnittansicht (Fig. 4b) des in Fign. 3a, 3b gezeigten Ventildruckknopfs.

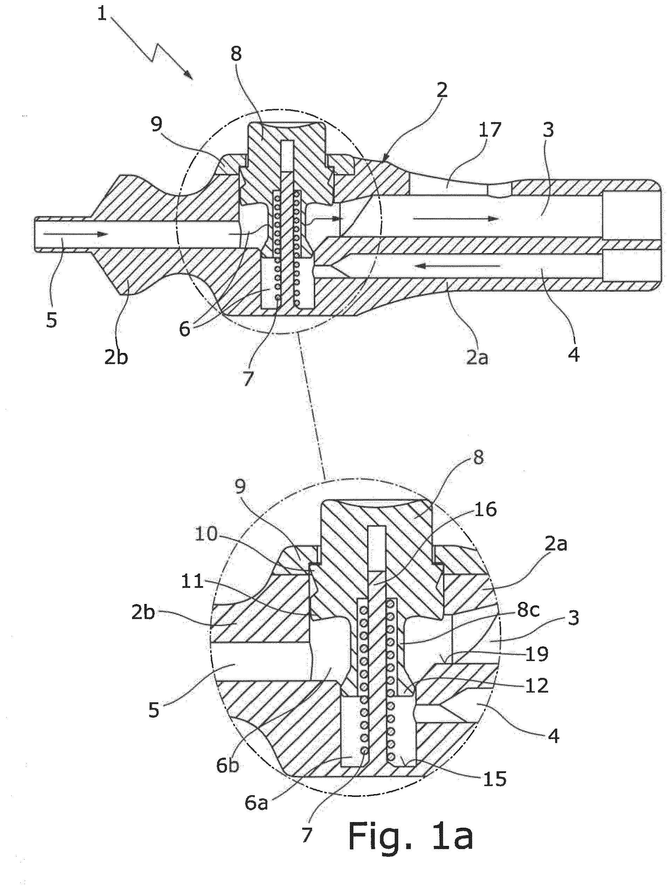

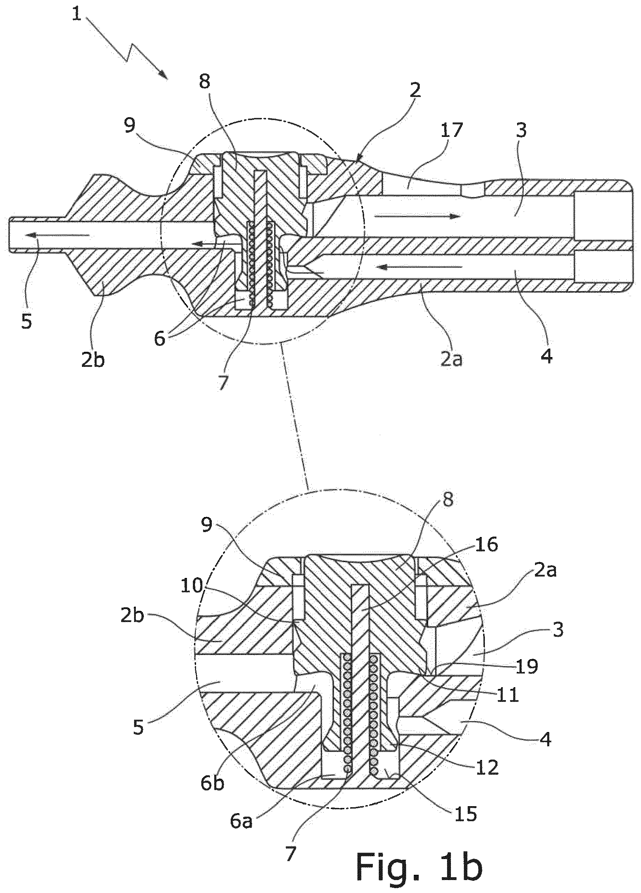

Der in **Fign. 1a****,** **1b** gezeigte Spülsauger **1** dient dazu, während einer medizinischen Operation entweder Flüssigkeit abzusaugen oder Spülflüssigkeit hinzuzugeben.

Der Spülsauger **1** umfasst ein einteilig gespritztes Kunststoff-Spülsaugergehäuse **2** mit einem Saugkanal **3,** einem Spülkanal **4** und einem Saugspülkanal **5** sowie mit einem Ventilraum **6,** in den die Kanäle 3-5 jeweils münden bzw. abgehen, und einen im Ventilraum 6 gegen die Kraft einer schraubenförmigen Rückstellfeder (Druckfeder) **7** verschiebbar geführten, manuell betätigbaren Ventildruckknopf **8,** der die Verbindung des Saugspülkanals 5 mit dem Saugkanal 3 und/oder dem Spülkanal 4 steuert.

Durch den Ventilraum 6 ist das Spülsaugergehäuse 2 unterteilt in ein distales Gehäuseende **2a,** in dem der Saugkanal 3 und der Spülkanal 4 parallel zueinander verlaufen, und ein proximales Gehäuseende **2b,** in dem der Saugspülkanal 5 verläuft. Der Ventilraum 6 ist durch eine einseitig (in Fign. 1a, 1b oben) offene Stufenbohrung gebildet, die einen unteren Ventilraum **6a** mit kleinerem Durchmesser und eine oberen Ventilraum **6b** mit größerem Durchmesser aufweist. Jeweils auf der rechten Seite des Ventilraums 6 geht der Saugkanal 3 vom oberen Ventilraum 6b ab und mündet der Spülkanal 4 in den unteren Ventilraum 6a. Zwischen Saug- und Spülkanal 3, 4, aber auf der linken Seite des Ventilraums 6 mündet der Saugspülkanal 5 in den oberen Ventilraum 6b. Der einstückig aus Silikon (z.B. Silikonkautschuk oder Silikonelastomere) gebildete Ventildruckknopf 8 ist von oben in den Ventilraum 6 eingesetzt und darin mittels eines ringförmigen Deckels **9** aus Kunststoff gesichert, der nach Montage des Ventildruckknopfs 8 und der Rückstellfeder 7 mit dem Spülsaugergehäuse 2 verbunden, insbesondere ultraschallverschweißt ist.

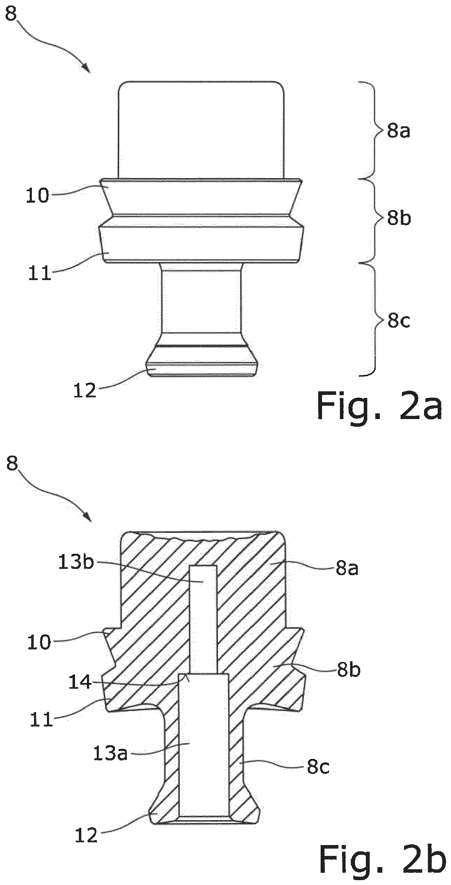

Wie in **Fign. 2a, 2b** gezeigt, weist der Ventildruckknopf 8 oben einen zylindrischen Betätigungsabschnitt **8a,** einen zylindrischen Mittelabschnitt **8b** mit zwei radial nach außen vorstehenden, ringförmigen Dichtlippen **10, 11** und unten einen zylindrischen Schaft **8c** mit einer radial nach außen vorstehenden, ringförmigen Dichtlippe **12** auf. Die drei Dichtlippen 10, 11, 12 sind am Ventildruckknopf 8 einstückig angeformt. Die Außenseite der Dichtlippe 12 verläuft nach unten leicht konisch. Der Außendurchmesser der am Mittelabschnitt 8b angeformten Dichtlippen 10, 11 ist größer als der Außendurchmesser des Betätigungsabschnitts 8a, der wiederum größer als der Außendurchmesser der am Schaft 8c angeformten Dichtlippe 12 ist. Im Ventildruckknopf 8 verläuft eine nach unten offene, gestufte Sackbohrung, die einen unteren Bohrungsabschnitt **13a** mit größeren Bohrungsdurchmesser, einen oberen Bohrungsabschnitt **13b** mit kleinerem Bohrungsdurchmesser und dazwischen - im Mittelabschnitt 8b - einen Ringabsatz **14** aufweist.

Vom Gehäuseboden **15** des Ventilraums 6 steht ein zylindrischer Führungsvorsprung **16** hoch, auf dem sowohl die Rückstellfeder 7 als auch der Ventildruccknopf 8 verschiebbar geführt sind. Dazu entsprechen der kleinere Bohrungsdurchmesser des oberen Bohrungsabschnitts 13b dem Durchmesser des Führungsvorsprungs 16 und der größere Bohrungsdurchmesser des unteren Bohrungsabschnitts 13a dem Außendurchmesser der Rückstellfeder 7. Die Rückstellfeder 7 ist einenends am Gehäuseboden 15 and anderenends am Ringabsatz 14 des Ventildruckknopfs 8 abgestützt. Aufgrund der zylindrischen bzw. rotationssymmetrischen Form des Ventildruckknopfs 8 kann die Kraft durch die Betätigung und durch die Rückstellfeder 7 optimal verteilt werden. Zudem muss bei der Montage des Ventildruckknopfs 8 auf keine Einbaudrehrichtung geachtet werden.

Durch die Rückstellfeder 7 ist der Ventildruckknopf 8 nach oben in die in Fig. 1a gezeigte Ventilausgangsstellung vorgespannt, in der die am Mittelabschnitt 8b angeformte Dichtkante 10 am Deckel 9 anliegt.

In der Ventilausgangsstellung ist die Verbindung des Saugspülkanals 5 zum Saugkanal 3 durch den vom radial nach innen zurückgesetzten Schaft 8c gebildeten Ringraum freigegeben und die Verbindung des Spülkanals 4 zum Saugspülkanal 5 durch die unten am Schaft 8c angeformte Dichtlippe 12 gesperrt, die oberhalb des Spülkanals 4 den unteren Ventilraum 6a vom oberen Ventilraum 6b absperrt. Der Schaftdurchmesser sollte so klein wie möglich sein, damit der Ringraum möglichst groß und somit kein Hindernis bildet, das evtl. zu Verstopfungen führen kann. Das im unteren Ventilraum 6a unter Druck stehende Spülwasser drückt einerseits von unten gegen die Dichtlippe 12, die dadurch elastisch hochgebogen wird, bis ihre ursprünglich konische Außenseite nunmehr flächig an der Wandung des unteren Ventilraums 6a angepresst ist, und füllt andererseits auch den unteren Bohrungsabschnitt 13a des Ventildruckknopfs 8, wodurch der Schaft 8c aufgespreizt und die Dichtlippe 12 gegen die Wandung des unteren Ventilraums 6a gepresst wird. Je nach gewünschter Saugleitung wird eine nach außen offene Saugregulierungsöffnung **17** des Saugkanals 3 vom Bediener unterschiedlich weit freigegeben. Der Bediener hält den Spülsauger 1 in der Hand und kann mit dem Daumen oder Zeigefinger den Ventildruckknopf 8 betätigen und/oder die Saugregulierungsöffnung 17 unterschiedlich weit abdecken.

Durch Niederdrücken des aus dem Spülsaugergehäuse 2 vorstehenden Betätigungsabschnitts 8a kann der Ventildruckknopf 8 aus der Ventilausgangsstellung bis in die in Fig. 1b gezeigte Ventilendstellung geschoben werden, in der die am Mittelabschnitt 8b angeformte Dichtlippe 11 auf einem Absatz **19** des Ventilraums 6 aufliegt. In der Ventilendstellung ist die unten am Schaft 8c angeformte Dichtlippe 12 bis unterhalb des in den unteren Ventilraum 6a mündenden Spülkanals 4 verschoben, so dass die Verbindung des Spülkanals 4 zum Saugspülkanal 5 durch den vom radial nach innen zurückgesetzten Schaft 8c gebildeten Ringraum freigegeben und die Verbindung des Saugspülkanals 5 zum Saugkanal 3 durch die am Mittelabschnitt 8b angeformte Dichtlippe 11 entweder vollständig gesperrt, wie in Fig. 1b gezeigt, oder bei leichter Betätigung gedrosselt ist. Bei dem gezeigten Ausführungsbeispiel kann in den Ventilendstellungen entweder gespült oder abgesaugt werden.

Nach Loslassen wird der Ventildruckknopf 8 durch die Rückstellfeder 7 wieder zurück in die Ventilausgangsstellung gedrückt und dadurch die Verbindung zwischen Spülkanal 4 und Saugspülkanal 5 wieder verschlossen.

Dadurch, dass der Ringabsatz 14, an dem die Rückstellfeder 7 abgestützt ist, im breiteren Mittelbereich 8b angeordnet ist, wird der Schaft 8c durch die Federkraft der Rückstellfeder 7 nicht verformt und somit die Dichtwirkung der Dichtlippe 12 nicht negativ beeinflusst. Der Schaftdurchmesser ist so gewählt, dass die Schaftwandung genügend Stabilität aufweist und beim Absaugen kein Hindernis bildet.

In jeder Stellung des Ventildruckknopfes 8 dichtet die am Mittelabschnitt 8b oben angeformte Dichtlippe 10 den Mittelabschnitt 8b gegenüber dem oberen Ventilraum 6b ab, so dass kein Sekret oder Spülwasser aus dem oberen Ventilraum 6b nach außen und an die Hände des Bedieners gelangen kann.

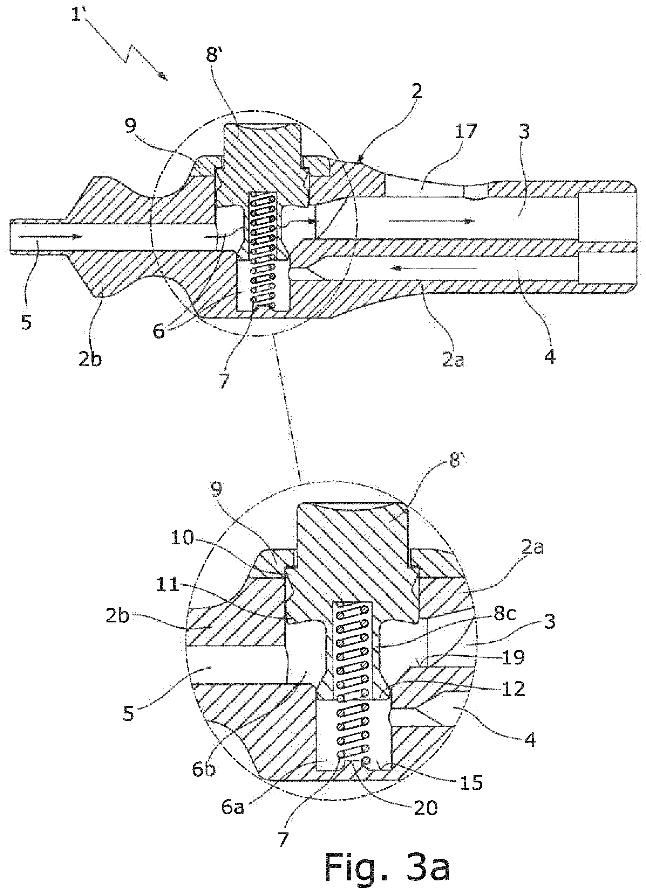

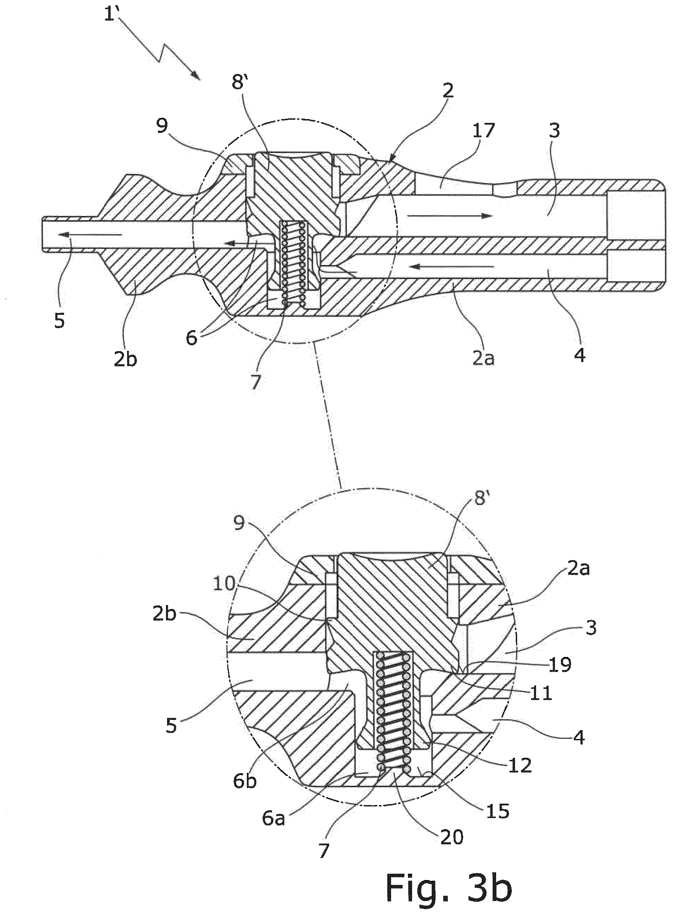

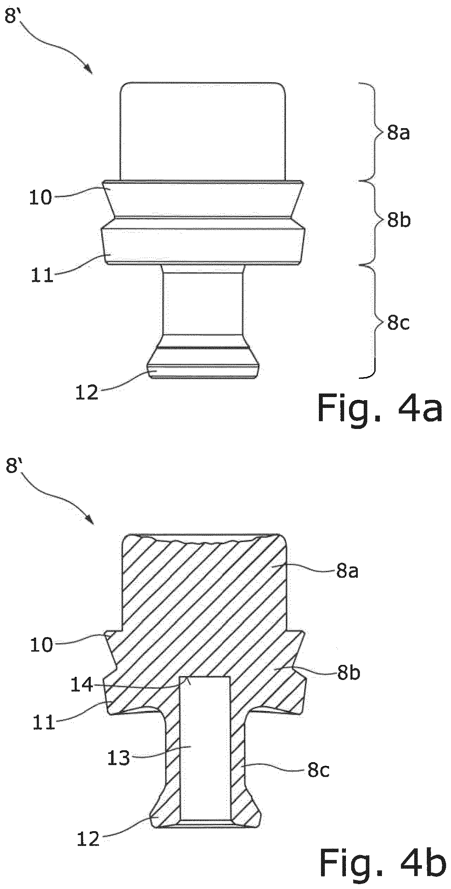

Vom Spülsauger 1 unterscheidet sich der in den **Fign. 3a****,** **3b** gezeigte Spülsauger **1'** dadurch, dass hier vom Gehäuseboden 15 kein Führungsvorsprung für den Ventildruckknopf **8'** hochsteht und der in **Fign. 4a, 4b** gezeigte Ventildruckknopf 8' eine zum Gehäuseboden 15 des Ventilraums 6 offene Bohrung 13 mit einem Bohrungsgrund 14 aufweist, an dem die Rückstellfeder 7 abgestützt ist. Das andere Ende der Rückstellfeder 7 ist an einem kleinen Vorsprung **20** des Gehäusebodens 15 lagefixiert.

## Patentansprüche

1. Spülsauger (1; 1') aufweisend ein Spülsaugergehäuse (2) mit einem Saugkanal (3), einem Spülkanal (4) und einem Saugspülkanal (5) sowie mit einem Ventilraum (6), in den die Kanäle (3-5) jeweils münden bzw. abgehen, und einen einzigen, im Ventilraum (6) gegen die Kraft einer Rückstellfeder (7) verschiebbar geführten, manuell betätigbaren Ventildruckknopf (8; 8'), der einen aus dem Spülsaugergehäuse (2) vorstehenden Betätigungsabschnitt (8a) sowie mindestens einen Dichtungsabschnitt (10, 11, 12) zum Steuern der Verbindung des Saugspülkanals (5) mit dem Saugkanal (3) und/oder dem Spülkanal (4) aufweist,
**dadurch gekennzeichnet,**
**dass** der Ventilraum (6) gestuft mit einem dem Betätigungsabschnitt (8a) zugewandten, ersten Ventilraum (6b) mit größerem Durchmesser, in den der Saugkanal (3) und der Saugspülkanal (5) münden bzw. abgehen, und mit einem dem Betätigungsabschnitt (8a) abgewandten, zweiten Ventilraum (6a) mit kleinerem Durchmesser, in den der Spülkanal (4) mündet, ausgebildet ist, und dass der Ventildruckknopf (8; 8') einschließlich des mindestens einen Dichtungsabschnitts (10, 11, 12) einstückig aus einem gummielastischen Material oder aus Silikon gebildet ist.

2. Spülsauger nach Anspruch 1, **dadurch gekennzeichnet, dass** der Ventildruckknopf (8') eine zum Gehäuseboden (15) des Ventilraums (6) offene Bohrung (13) mit einem Absatz (14) aufweist, an dem die Rückstellfeder (7) abgestützt ist.

3. Spülsauger nach Anspruch 1, **dadurch gekennzeichnet, dass** von einem Gehäuseboden (15) des Ventilraums (6) ein Führungsvorsprung (16) hochsteht, auf dem sowohl der Ventildruckknopf (8) als auch die Rückstellfeder (7) geführt sind.

4. Spülsauger nach Anspruch 3, **dadurch gekennzeichnet, dass** der Ventildruckknopf (8) eine zum Gehäuseboden (15) des Ventilraums (6) offene, gestufte Sackbohrung (13a, 13b) mit einem kleineren und einem größeren Bohrungsdurchmesser sowie mit einem Absatz (14) aufweist, an dem die Rückstellfeder (7) abgestützt ist, wobei der kleinere Bohrungsdurchmesser dem Durchmesser des Führungsvorsprungs (16) und der größere Bohrungsdurchmesser mindestens dem Außendurchmesser der Rückstellfeder (7) entspricht.

5. Spülsauger nach einem der Ansprüche 2 bis 4, **dadurch gekennzeichnet, dass** der Absatz (14) in einem Mittelabschnitt (8b) oder in einem Betätigungsabschnitt (8a) des Ventildruckknopfs (8; 8') angeordnet ist.

6. Spülsauger nach einem der vorhergehenden Ansprüche, **dadurch gekennzeichnet, dass** der Ventildruckknopf (8; 8') einen Betätigungsabschnitt (8a), einen zylindrischen Schaft (8c) und dazwischen einen zylindrischen Mittelabschnitt (8b) aufweist, gegenüber dem der Betätigungsabschnitt (8a) und der Schaft (8c) jeweils radial nach innen zurückversetzt sind.

7. Spülsauger nach Anspruch 6, **dadurch gekennzeichnet, dass** am Schaft (8c) ein Dichtungsabschnitt (12) einstückig angeformt ist, welcher die Verbindung des Saugspülkanals (5) mit dem Spülkanal (4) in einer ersten Ventilstellung des Ventildruckknopfs (8) sperrt und in einer zweiten Ventilstellung des Ventildruckknopfs (8) freigibt.

8. Spülsauger nach Anspruch 7, **dadurch gekennzeichnet, dass** am Mittelabschnitt (8b) ein Dichtungsabschnitt (11) einstückig angeformt ist, welcher die Verbindung des Saugspülkanals (5) mit dem Saugkanal (3) in der ersten Ventilstellung des Ventildruckknopfs (8) freigibt und in der zweiten Ventilstellung sperrt.

9. Spülsauger nach Anspruch 8, **dadurch gekennzeichnet, dass** der Außendurchmesser des am Schaft (8c) angeformten Dichtungsabschnitts (12) kleiner als der Außendurchmesser des am Mittelabschnitt (8b) angeformten Dichtungsabschnitts (11) ist.

10. Spülsauger nach Anspruch 8 oder 9, **dadurch gekennzeichnet, dass** in der zweiten Ventilstellung des Ventildruckknopfs (8) der am Mittelabschnitt (8b) angeformte Dichtungsabschnitt (11) an einem Endanschlag (15) des Spülsaugergehäuses (2) anliegt.

11. Spülsauger nach einem der Ansprüche 6 bis 10, **dadurch gekennzeichnet, dass** der Durchmesser des Schaftes (8c) kleiner als der Durchmesser des zweiten Ventilraums (6a) ist.

12. Spülsauger nach einem der Ansprüche 7 bis 11, **dadurch gekennzeichnet, dass** der am Schaft (8c) angeformte Dichtungsabschnitt (12) in der ersten Ventilstellung des Ventildruckknopfs (8; 8') auf der dem ersten Ventilraum (6b) zugewandten Seite des in den zweiten Ventilraum (6b) mündenden Spülkanals (4) und in der zweiten Ventilstellung auf der dem ersten Ventilraum (6b) abgewandten Seite des in den zweiten Ventilraum (6a) mündenden Spülkanals (4) angeordnet ist.

13. Spülsauger nach einem der Ansprüche 7 bis 12, **dadurch gekennzeichnet, dass** der Ventildruckknopf (8) einen Absatz aufweist, der in der ersten Ventilstellung am Spülsaugergehäuse (2) oder an einem mit dem Spülsaugergehäuse (2) verbundenen Deckel (9) anliegt.

14. Spülsauger nach einem der vorhergehenden Ansprüche, **dadurch gekennzeichnet, dass** der Saugkanal (3) eine nach außen offene Saugregulierungsöffnung (17) aufweist.

## Claims

1. Flushing and aspirating device (1; 1'), comprising a device housing (2) with an aspirating channel (3), a flushing channel (4) and a flushing and aspirating channel (5) and with a valve chamber (6) in which the channels (3-5) empty or emerge therefrom, respectively, and comprising one single manually operable valve push button (8; 8') which is movably guided in the valve chamber (6) against the force of a reset spring (7) and which has an actuating section (8a) protruding from the device housing (2) and at least one sealing section (10, 11, 12) for controlling the connection of the flushing and aspirating channel (5) to the aspirating channel (3) and/or the flushing channel (4),
**characterized in that**
the valve chamber (6) is configured stepped, with a first valve chamber (6b) facing the actuating section (8a) and having a larger diameter, in which the aspirating channel (3) and the flushing and aspirating channel (5) empty or emerge therefrom, and with a second valve chamber (6a) facing away from the actuating section (8a) and having a smaller diameter, in which the flushing channel (4) empties, and that the valve push button (8; 8') including the at least one sealing section (10, 11, 12) is formed as a single piece of an elastic rubber material or of silicone.

2. Flushing and aspirating device according to claim 1, **characterized in that** the valve push button (8') comprises a bore (13), which is open towards the housing bottom (15) of the valve chamber (6), with a shoulder (14), against which the reset spring (7) is braced.

3. Flushing and aspirating device according to claim 1, **characterized in that** a guiding projection (16) rises up from a housing bottom (15) of the valve chamber (6), on which both the valve push button (8) and the reset spring (7) are guided.

4. Flushing and aspirating device according to claim 3, **characterized in that** the valve push button (8) comprises a stepped blind bore (13a, 13b), which is open towards the housing bottom (15) of the valve chamber (6), having a smaller and a larger bore diameter and having a shoulder (14), against which the reset spring (7) is braced, the smaller bore diameter corresponding to the diameter of the guiding projection (16) and the larger bore diameter corresponding at least to the outer diameter of the reset spring (7).

5. Flushing and aspirating device according to one of claims 2 to 4, **characterized in that** the shoulder (14) is situated in a middle section (8b) or in an actuating section (8a) of the valve push button (8; 8').

6. Flushing and aspirating device according to one of the preceding claims, **characterized in that** the valve push button (8; 8') comprises an actuating section (8a), a cylindrical shaft (8c) and a cylindrical middle section (8b) in between, relative to which the actuating section (8a) and the shaft (8c) are respectively set back radially inwards.

7. Flushing and aspirating device according to claim 6, **characterized in that** on the shaft (8c), a sealing section (12) is formed as a single piece, which blocks the connection of the flushing and aspirating channel (5) to the flushing channel (4) in a first valve position of the valve push button (8) and releases it in a second valve position of the valve push button (8).

8. Flushing and aspirating device according to claim 7, **characterized in that** on the middle section (8b), a sealing section (11) is formed as a single piece, which releases the connection of the flushing and aspirating channel (5) to the aspirating channel (3) in the first valve position of the valve push button (8) and blocks it in the second valve position.

9. Flushing and aspirating device according to claim 8, **characterized in that** the outer diameter of the sealing section (12) formed on the shaft (8c) is smaller than the outer diameter of the sealing section (11) formed on the middle section (8b).

10. Flushing and aspirating device according to claim 8 or 9, **characterized in that** in the second valve position of the valve push button (8) the sealing section (11) formed on the middle section (8b) rests against an end stop (15) of the device housing (2).

11. Flushing and aspirating device according to one of the claims 6 to 10, **characterized in that** the diameter of the shaft (8c) is smaller than the diameter of the second valve chamber (6a).

12. Flushing and aspirating device according to one of the claims 7 to 11, **characterized in that** the sealing section (12) formed on the shaft (8c) in the first valve position of the valve push button (8; 8') is situated at the side facing the first valve chamber (6b) of the flushing channel (4) emptying into the second valve chamber (6a) and in the second valve position it is situated at the side facing away from the first valve chamber (6b) of the flushing channel (4) emptying into the second valve chamber (6a).

13. Flushing and aspirating device according to one of the claims 7 to 12, **characterized in that** the valve push button (8) comprises a shoulder, which in the first valve position rests against the device housing (2) or against a cover (9) connected to the device housing (2).

14. Flushing and aspirating device according to one of the preceding claims, **characterized in that** the aspirating channel (3) comprises an outwardly open aspiration regulating opening (17).

## Revendications

1. Dispositif (1 ; 1') de rinçage et d'aspiration, comportant un boîtier (2) muni d'un canal d'aspiration (3), d'un canal de rinçage (4) et d'un canal (5) d'aspiration et de rinçage, ainsi que d'une chambre de distribution (6) dans laquelle lesdits canaux (3-5) débouchent, ou dont ils partent respectivement, et un unique bouton poussoir (8 ; 8') de soupape qui peut être actionné à la main, est guidé à coulissement dans ladite chambre de distribution (6) en opposition à la force d'un ressort de rappel (7), et comprend un tronçon d'actionnement (8a) faisant saillie au-delà du boîtier (2) dudit dispositif de rinçage et d'aspiration, ainsi qu'au moins un tronçon d'étanchement (10, 11, 12), en vue de commander la liaison du canal (5) d'aspiration et de rinçage avec le canal d'aspiration (3) et/ou le canal de rinçage (4),
**caractérisé par le fait**
**que** la chambre de distribution (6) est de réalisation étagée comprenant un premier compartiment de distribution (6b) de plus fort diamètre, pointant vers le tronçon d'actionnement (8a), et dans lequel le canal d'aspiration (3) et le canal (5) d'aspiration et de rinçage débouchent, ou dont ils partent respectivement, et un second compartiment de distribution (6a) de diamètre moindre, pointant à l'opposé dudit tronçon d'actionnement (8a), et dans lequel le canal de rinçage (4) débouche ; et par le fait que le bouton poussoir (8 ; 8') de soupape est ménagé d'un seul tenant, y compris le tronçon d'étanchement (10, 11, 12) à présence minimale, en un matériau doué de l'élasticité du caoutchouc, ou en du silicone.

2. Dispositif de rinçage et d'aspiration selon la revendication 1, **caractérisé par le fait que** le bouton poussoir (8') de soupape comporte un alésage (13) ouvert vers le fond (15) de l'enceinte de la chambre de distribution (6), et présentant un décrochement (14) contre lequel le ressort de rappel (7) est en appui.

3. Dispositif de rinçage et d'aspiration selon la revendication 1, **caractérisé par le fait qu'**une protubérance de guidage (16), sur laquelle le bouton poussoir (8) de soupape et le ressort de rappel (7) sont l'un et l'autre guidés, se dresse à partir d'un fond (15) de l'enceinte de la chambre de distribution (6).

4. Dispositif de rinçage et d'aspiration selon la revendication 3, **caractérisé par le fait que** le bouton poussoir (8) de soupape est muni d'un alésage borgne étagé (13a, 13b) ouvert vers le fond (15) de l'enceinte de la chambre de distribution (6) et présentant un diamètre moindre et un diamètre plus fort, ainsi qu'un décrochement (14) contre lequel le ressort de rappel (7) est en appui, sachant que le diamètre moindre de l'alésage correspond au diamètre de la protubérance de guidage (16), et que le plus fort diamètre dudit alésage correspond au moins au diamètre extérieur dudit ressort de rappel (7).

5. Dispositif de rinçage et d'aspiration selon l'une des revendications 2 à 4, **caractérisé par le fait que** le décrochement (14) est situé dans une région médiane (8b) ou sur un tronçon d'actionnement (8a) du bouton poussoir (8 ; 8') de soupape.

6. Dispositif de rinçage et d'aspiration selon l'une des revendications précédentes, **caractérisé par le fait que** le bouton poussoir (8 ; 8') de soupape comprend un tronçon d'actionnement (8a), un fût cylindrique (8c) et, entre ces derniers, une région médiane cylindrique (8b) par rapport à laquelle ledit tronçon d'actionnement (8a) et ledit fût (8c) sont respectivement en retrait vers l'intérieur, dans le sens radial.

7. Dispositif de rinçage et d'aspiration selon la revendication 6, **caractérisé par le fait qu'**un tronçon d'étanchement (12), ménagé d'un seul tenant avec le fût (8c), interdit la liaison du canal (5) d'aspiration et de rinçage avec le canal de rinçage (4), dans une première position de distribution du bouton poussoir (8) de soupape, et l'autorise dans une seconde position de distribution dudit bouton poussoir (8) de soupape.

8. Dispositif de rinçage et d'aspiration selon la revendication 7, **caractérisé par le fait qu'**un tronçon d'étanchement (11), ménagé d'un seul tenant avec la région médiane (8b), autorise la liaison du canal (5) d'aspiration et de rinçage avec le canal d'aspiration (3), dans la première position de distribution du bouton poussoir (8) de soupape, et l'interdit dans la seconde position de distribution.

9. Dispositif de rinçage et d'aspiration selon la revendication 8, **caractérisé par le fait que** le diamètre extérieur du tronçon d'étanchement (12), faisant corps avec le fût (8c), est plus petit que le diamètre extérieur du tronçon d'étanchement (11) faisant corps avec la région médiane (8b).

10. Dispositif de rinçage et d'aspiration selon la revendication 8 ou 9, **caractérisé par le fait que**, dans la seconde position de distribution du bouton poussoir (8) de soupape, le tronçon d'étanchement (11), faisant corps avec la région médiane (8b), est en applique contre une butée d'extrémité (15) du boîtier (2) dudit dispositif de rinçage et d'aspiration.

11. Dispositif de rinçage et d'aspiration selon l'une des revendications 6 à 10, **caractérisé par le fait que** le diamètre du fût (8c) est plus petit que le diamètre du second compartiment de distribution (6a).

12. Dispositif de rinçage et d'aspiration selon l'une des revendications 7 à 11, **caractérisé par le fait que** le tronçon d'étanchement (12) faisant corps avec le fût (8c) est situé, dans la première position de distribution du bouton poussoir (8 ; 8') de soupape, du côté du canal de rinçage (4) qui pointe vers le premier compartiment de distribution (6b), lequel canal débouche dans ledit premier compartiment de distribution (6b); et est situé, dans la seconde position de distribution, du côté dudit canal de rinçage (4) qui pointe à l'opposé dudit premier compartiment de distribution (6b), lequel canal débouche dans le second compartiment de distribution (6a).

13. Dispositif de rinçage et d'aspiration selon l'une des revendications 7 à 12, **caractérisé par le fait que** le bouton poussoir (8) de soupape est pourvu d'un décrochement en applique, dans la première position de distribution, contre le boîtier (2) dudit dispositif de rinçage et d'aspiration, ou contre un couvercle (9) relié audit boîtier (2) du dispositif de rinçage et d'aspiration.

14. Dispositif de rinçage et d'aspiration selon l'une des revendications précédentes, **caractérisé par le fait que** le canal d'aspiration (3) comporte un orifice (17) régulateur d'aspiration, ouvert vers l'extérieur.
